# EUROPEAN PATENT APPLICATION

(11) **EP 4 099 020 A1**
(43) Date of publication of application: **07.12.2022**
(21) Application number: 20916742.8
(22) Date of filing: 27.11.2020
(51) Int. Cl.: G01N 35/08, G01N 33/483, G01N 33/543, G01N 21/05

(54) **FLOW-CELL AND AUTOMATED ANALYSIS DEVICE**

(30) Priority: 30.01.2020 JP 2020013488
(71) Applicant: Hitachi High-Tech Corporation, Minato-ku Tokyo 105-6409 (JP)
(72) Inventor: NEGISHI, Yoshinori, Tokyo 100-8280 (JP); OGUCHI, So, Tokyo 105-6409 (JP); SUGIMURA, Yoshiaki, Tokyo 105-6409 (JP); KAJIHARA, Yuri, Tokyo 105-6409 (JP)
(74) Representative: MERH-IP Matias Erny Reichl Hoffmann Patentanwälte PartG mbB
(86) International application number: PCT/JP2020/044257
(87) International publication number: WO 2021/152987

(57) **Abstract**

Provided are a flow cell and an automatic analysis device which are excellent in the long-term stability of the flow path shape of the flow cell and can improve the reproducibility of analysis.

The flow cell (6) of the present invention includes a base (18) including an inlet and outlet (35, 36) for a flow path of a reaction solution containing an analyte; an electrode (16A, 16B) for applying a voltage to the analyte; a light receiving window (22) made of a member that transmits light emitted from the analyte by the voltage applied on the electrode, a gasket (18A) provided between the base (18) and the light receiving window (22), and a flow chamber (17) surrounded by the base (18), the light receiving window (22) and the gasket (18A), in which the gasket (18A) has a deformation amount of 0 mm or more and 0.2 mm or less in a chemical immersion test, and the surface adhesive force of 14 N/cm² or more and 40 N/cm² or less in a surface adhesion evaluation test.

## Description

### Technical Field

The present invention relates to a flow cell and an automatic analysis device.

### Background Art

An immunoassay utilizing a reaction between an antigen and an antibody is carried out, for example, by the following procedures. A reagent, which contains an antibody-coated magnetic particle and an antibody bound to a luminescent labelling substance, is mixed, in a reaction vessel, with a sample containing a substance (antigen) as an analysis target. According to an antigen-antibody reaction, with the antibody on a surface of the magnetic particle and the antibody bound to the luminescent labelling substance, the analysis target substance is captured and immobilized on the surface of the magnetic particle, and an immune complex is formed. A sample solution containing the prepared immune complex is passed through a flow cell in which a working electrode and a counter electrode are exposed to the inside of a flow path, and at the same time, a permanent magnet is brought into contact with an outer wall of the flow cell to apply a magnetic field on the working electrode. Then, the magnetic particle in the immune complex is captured on the working electrode under an influence of the magnetic field, and a liquid phase flows out of the flow cell. After a reaction solution passes through the flow cell, the inside of the flow cell is replaced with a buffer solution containing a luminescence inducing substance. Thereafter, the permanent magnet is separated from the flow cell, and when a voltage is applied between the working electrode and the counter electrode, the immune complex on the working electrode emits light (electrochemical luminescence). By detecting luminescence using a photodetector such as a photomultiplier tube, a target substance concentration in the sample can be quantified based on an amount of luminescence.

PTL 1 describes an example of an immunoassay device using a magnetic particle. PTL 2 discloses a flow-through type device that detects a chemical substance and an electrochemical luminescence immunoassay device using the flow-through type device. A gasket using a rubber material is used to form a flow path of a flow-through type cell (flow cell). It is shown that a fluoro rubber having good chemical resistance is suitable for the rubber material used in a portion to be in contact with a solution.

### Citation List

### Patent Literature

PTL 1: JP-A-8-146002
PTL 2: JP-A-11-118706

### Summary of Invention

### Technical Problem

A capture state of the immune complex on the working electrode in the reaction solution affects detection sensitivity and reproducibility of measurement. The capture state of the immune complex on the working electrode is affected by a flow of a fluid in the flow cell. Therefore, when the flow cell is repeatedly used, long-term stability of a flow path shape of the flow cell is important.

PTL 1 discloses an automatic analysis device including a flow cell type detector, but there is no suggestion and disclosure regarding long-term flow path shape stability of the flow cell. In a method of forming a flow path of a flow cell by using the gasket made of the fluoro rubber described in PTL 2, a predetermined flow path shape can be easily formed. However, even in the flow cell using the gasket using the fluoro rubber selected from a viewpoint of the chemical resistance, deformation of the gasket caused by repeated use, that is, deformation in the flow path shape occurs.

In view of the above circumstances, an object of the invention is to provide a flow cell and an automatic analysis device which are excellent in long-term stability of a flow path shape of the flow cell and can improve reproducibility of analysis.

### Solution to Problem

According to an aspect of the invention for solving the above problems, there is provided a flow cell including: a base provided with an inlet and outlet of a flow path of a fluid containing an analyte, an electrode for applying a voltage to the analyte, a light receiving window made of a member that transmits light emitted from the analyte by the voltage applied on the electrode, a gasket provided between the base and the light receiving window, and a flow chamber surrounded by the base, the light receiving window and the gasket, in which the gasket has a deformation amount of 0 mm or more and 0.2 mm or less in the chemical immersion test defined in the following (1), and a surface adhesive force of 14 N/cm² or more and 40 N/cm² or less in the surface adhesion evaluation test defined in the following (2):
(1) Chemical immersion test: After immersion in 0.2 mol/L of potassium hydroxide solution at 40°C for 31 days, the amount of dimensional change is measured.
(2) Surface adhesion evaluation test: A lower surface of a test piece (10 mm square, 0.5 mm thick) is fixed to acrylic resin 1 (20 mm × 30 mm, 2 mm thick) with double-sided tape; acrylic resin 2 (20 mm × 60 mm, 2 mm thick) is placed on an upper surface of the test piece; a 2 kg weight is allowed to stand on the upper surface of the acrylic resin 2 for 1 minute; the acrylic resin 2 of the evaluation sample obtained by integrating the acrylic resin 1, the test piece, and the acrylic resin 2 is moved in a vertical direction; and the force at which the interface between the test piece and the acrylic resin 2 separates is measured with a force gauge.

According to another aspect of the invention for solving the above problems, there is provided an automatic analysis device including: a flow cell according to the first aspect of the invention; a unit configured to supply a fluid containing magnetic particles having a labeling substance to the flow cell; a voltage applying unit configured to apply a voltage to the electrode; a magnetic trapping unit configured to trap the magnetic particles supplied to the flow cell at a predetermined location by a magnetic field; and a light detection unit configured to detect light generated from the flow cell via the light receiving window, in which the gasket has a deformation amount of 0 mm or more and 0.2 mm or less in the chemical immersion test defined in the above (1), and the gasket has a surface adhesive force of 14 N/cm² or more and 40 N/cm² or less in the surface adhesion evaluation test defined in the above (2).

A more specific configuration of the invention is described in claims.

### Advantageous Effects of Invention

According to the invention, it is possible to provide a flow cell and an automatic analysis device which are excellent in long-term stability of a flow path shape of the flow cell and can improve reproducibility of analysis.

Problems, configurations, and effects other than those described above will be clarified by the following description of embodiments.

### Brief Description of Drawings

[Fig. 1] Fig. 1 is a diagram showing a schematic configuration of an automatic analysis device according to an embodiment of the present invention.
[Fig. 2] Fig. 2 is a longitudinal sectional view showing a configuration of a flow cell and a detection unit of Fig. 1.
[Fig. 3] Fig. 3 is a sectional view taken along line I-I of Fig. 2.
[Fig. 4] Fig. 4 is a sectional view taken along line II-II in Fig. 3.
[Fig. 5] Fig. 5 is a graph in which the deformation amount (chemical resistance) of the test samples and the surface adhesive force are plotted.

### Description of Embodiments

Hereinafter, embodiments of the invention will be described, and the invention is not limited to these descriptions.

### [Automatic Analysis Device]

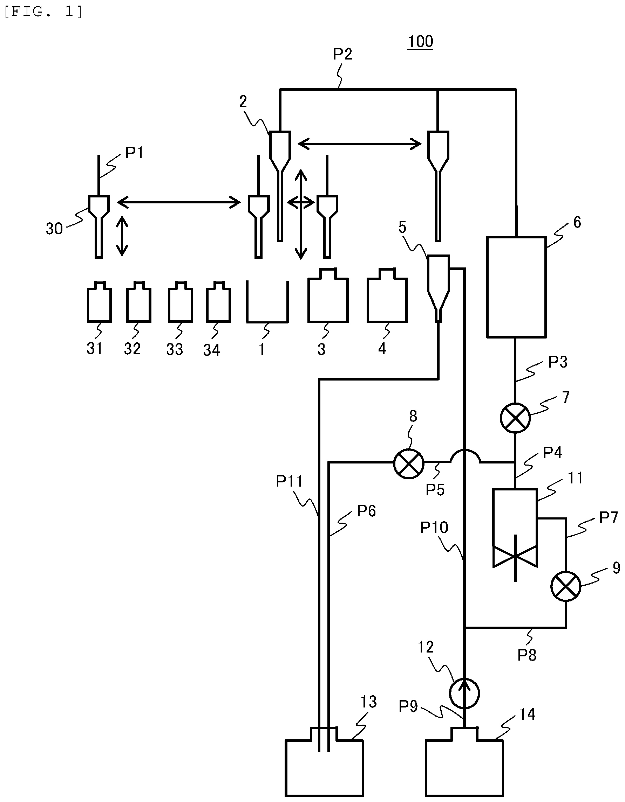

First, a configuration of an automatic analysis device will be described. Fig. 1 is a diagram showing a schematic configuration of the automatic analysis device according to an embodiment of the invention. In Fig. 1, an automatic analysis device 100 according to the present embodiment includes a sample bottle 31 that contains a sample, a bead bottle 32 that contains a bead solution that contains magnetic particles, a first reagent bottle 33 that contains a first reagent that binds the magnetic particles to a specific component in the sample, a second reagent bottle 34 that contains a second reagent having a labeling substance that labels a substance to be labeled that emits light in response to an electrochemical reaction and that binds with the specific component in the sample, a buffer solution bottle 3 that contains a buffer solution containing a substance for inducing electrochemical luminescence of the labeling substance, a cleaning liquid bottle 4 that contains a cleaning liquid, a vessel 1 for obtaining a suspension containing a reaction product, a sampling probe 30 that dispenses the sample, beads, the first reagent, the second reagent, and the buffer solution to the vessel 1, a shipper probe 2 that delivers the suspension of the vessel 1, a cleaning tank 5 in which a tip end portion of the shipper probe 2 is to be cleaned, a flow cell 6 into which the suspension delivered from the shipper probe 2 is introduced, a syringe 11 that aspirates and discharges the suspension, the cleaning liquid, and the buffer solution, a waste liquid bottle 13 that stores a waste liquid, a distilled water bottle 14 that stores distilled water, and a pump 12 that delivers the distilled water of the distilled water bottle 14 to the cleaning tank 5.

The sampling probe 30 includes a known pipetting mechanism, and is connected to a syringe (not shown) via a conduit P1. The shipper probe 2 is connected to the flow cell 6 via a conduit P2. The flow cell 6 is connected to the syringe 11 via a conduit P3, a first pinch valve 7, and a conduit P4. The conduit P4 is connected to the waste liquid bottle 13 via a conduit P5, a second pinch valve 8, and a conduit P6.

On the other hand, the distilled water bottle 14 is connected to the cleaning tank 5 via a conduit P9, the pump 12, and a conduit P10, and the cleaning tank 5 is connected to the waste liquid bottle 13 via a conduit P11. A conduit P8 branches from the middle of the conduit P10, and the conduit P8 is connected to the syringe 11 via a third pinch valve 9 and a conduit P7.

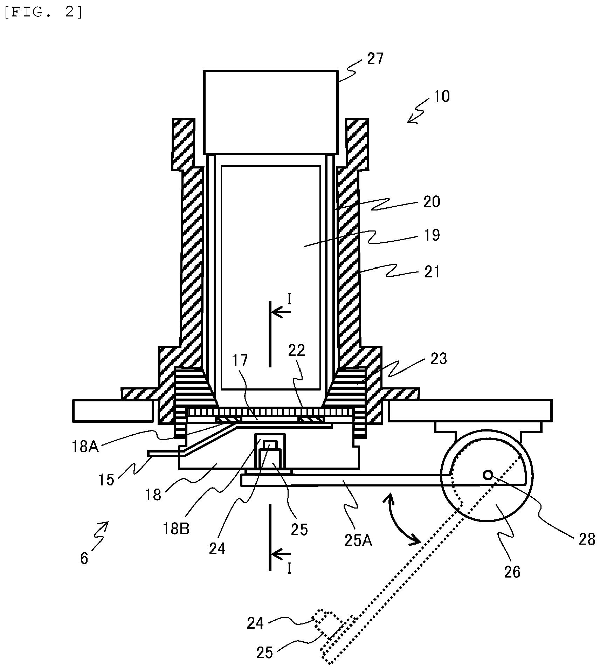

Next, a light detection unit 10 including the flow cell 6 constituting the automatic analysis device 100 will be described in detail. Fig. 2 is a longitudinal sectional view showing a configuration of the flow cell of Fig. 1 and a detection unit. The light detection unit 10 detects light generated in a flow chamber 17 of the flow cell 6 and transmitted through a light receiving window 22. A photomultiplier tube (PMT) 19 is used for receiving the light. As the photomultiplier tube, for example, a photomultiplier tube R1878 manufactured by Hamamatsu Photonics Co., Ltd. can be used. The photomultiplier tube 19 is covered with a shield tube 20 and accommodated in a PMT case 21 in order to prevent a decrease in multiplication efficiency caused by magnetism. A socket 27 is attached above the photomultiplier tube 19, a detection signal of the photomultiplier tube 19 is transmitted to a control device (not shown) via the socket 27, and light intensity is measured.

The flow cell 6 includes a base 18, and the light receiving window 22 positioned between the base 18 and the light detection unit 10, and the base 18, a gasket 18A, and the light receiving window 22 are integrated by a cell holder 23 to form the flow chamber 17.

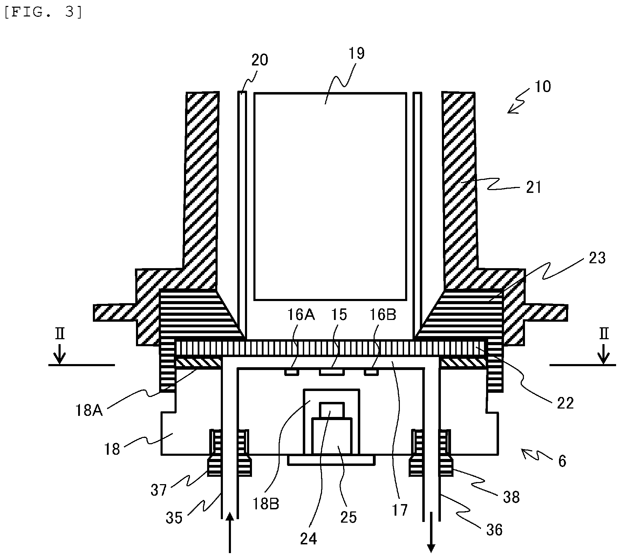

Fig. 3 is a sectional view taken along a line I-I of Fig. 2. As shown in Fig. 3, a flow path inlet 35 and a flow path outlet 36 (Fig. 3) are formed at both ends of the flow chamber 17, and the flow path inlet 35 and the flow path outlet 36 are connected to the conduits P2 and P3 (Fig. 1), respectively, via nipples 37 and 38 attached to the base 18. A working electrode 15 is embedded in the base 18, a pair of counter electrodes 16A and 16B are embedded in a symmetrical shape on the same plane on both sides of the working electrode 15, and both electrode surfaces are polished together with the base 18. One end of each of the working electrode 15 and the counter electrode 16 extends to the outside of the base 18, and is connected to a power supply and the control device (not shown).

A magnet 24 is positioned below the working electrode 15, and the magnet 24 is disposed in a recess 18B formed in the base 18 such that the magnet 24 approaches the working electrode 15 and applies a magnetic field to the working electrode 15. The magnet 24 is attached to a magnet holder 25, and the magnet holder 25 is attached to one end of a lever 25A (Fig. 2). As shown in Fig. 2, the other end of the lever 25A is attached to a stepping motor 26 and can rotate around a fulcrum 28. By operating the stepping motor 26, the magnet 24 can be driven between an operating position shown by a solid line in the recess 18B and a retracted position shown by a dotted line outside the recess 18B.

Next, an analysis method used by the immunoassay device configured as described above will be described with reference to Figs. 1 to 3. When the sample is serum, a component (analyte) to be analyzed by the immunoassay device is an antigen, a peptide hormone, a steroid hormone, a drug, a virus antibody, various tumor markers, an antibody, an antibody complex, a single protein, or the like.

An antibody is immobilized on a surface of magnetic particles used as a solid phase. The magnetic particles are formed by, for example, embedding a powder of a magnetic attractive material such as iron, iron oxide, nickel, cobalt, or chromium oxide in a matrix, and the matrix itself includes a wide range of substances including many synthetic and natural polymerizable substances (for example, cellulose, polyester, polystyrene, silica, dextran, albumin, and the like).

An immune complex containing an analyte (antigen) and a luminescent labeling substance is bound onto the magnetic particles by an immune reaction. The immune complex is introduced, in a form of suspension, into the flow cell 6 together with other coexisting substances in a reaction mixture.

In the following, as an example, a case where a sample derived from a biological fluid such as serum or urine containing thyroid stimulating hormone (TSH) is analyzed will be described. 50 µl of the sample in the sample bottle 31 that is derived from the biological fluid such as serum or urine containing the thyroid stimulating hormone (TSH), which is a specific component, 50 µl of a bead solution (an average particle diameter being 2.8 µm and a specific gravity being 1.4 in the present embodiment) in the bead bottle 32 that is obtained by dispersing the magnetic particles in a buffer solution, 50 µl of a first reagent in the first reagent bottle 33 that contains a TSH antibody of which an end is treated with biotin, 50 µl of a second reagent in the second reagent bottle 34 that contains a TSH antibody of which an end is treated with biotin and which is bonded to a labeling substance (Ru(bpy)₃, that is, ruthenium(II)tris(bipyridyl) in the present embodiment) that generates chemiluminescence by excitation, and 50 µl of a buffer solution in the buffer solution bottle 3 having a pH of around 7.4 and containing a substance (tripropylamine (TPA) in the present embodiment) that induces the excitation of the labeling substance are sequentially dispensed into the vessel 1 by the sampling probe 30 in a predetermined order. Here, since analysis is performed by a sandwich method, the bead solution, the first reagent, the sample, and the second reagent are dispensed in this order.

During a dispensing operation, an inside of the vessel 1 is kept at a constant temperature (37°C in this embodiment) and stirring is performed by a vibrating device (not shown) to proceed the reaction, and heat retention and stirring are continued for a certain period of time (15 minutes in this embodiment) after the dispensing operation. Accordingly, a suspension containing a reaction product in which the magnetic particles, the first reagent, the TSH in the sample, and the second reagent are bound is generated in the vessel 1.

Next, the suspension in the vessel 1 is introduced into the flow chamber 17 of the flow cell 6. This operation is performed as follows. First, in Fig. 2, the stepping motor 26 is driven to move the magnet 24 to the operating position indicated by the solid line in Fig. 2. Next, in Fig. 1, the first pinch valve 7 is opened, and the second pinch valve 8 and the third pinch valve 9 are closed. In this state, first, the shipper probe 2 is horizontally moved to above the vessel 1 and then moved downward by a drive device (not shown), and the tip end portion of the shipper probe 2 is inserted into the suspension in the vessel 1.

Next, after 200 µl of suspension of 250 µl of suspension containing the reaction product in the vessel 1 is aspirated into the shipper probe 2 by the syringe 11, the shipper probe 2 is moved upward to move the tip end portion thereof out of the suspension, and then the suspension in the shipper probe 2 is aspirated again by the syringe 11. This suction causes the 200 µl of suspension to flow through the flow chamber 17 via the conduit P2. At this time, an operation of the syringe 11 is controlled such that the suspension flows through the flow chamber 17 at a linear velocity of 50 mm/s from the flow path inlet 35. When the suspension reaches above the working electrode 15, only the reaction product and unreacted magnetic particles are captured above the working electrode 15 by a magnetic field locally formed by the magnet 24, and the unreacted first reagent and second reagent pass through the flow chamber 17 and are aspirated toward the syringe 11. Accordingly, all the reaction products contained in the 200 µl of suspension are collected in the flow chamber 17.

Next, the shipper probe 2 is horizontally moved to above the cleaning tank 5 and then moved downward, and the tip end portion thereof is positioned in the cleaning tank 5. In this state, the pump 12 is driven, and the distilled water in the distilled water bottle 14 is discharged into the cleaning tank 5 via the conduit P9, the pump 12, and the conduit P10, and the outside of the tip end portion of the inserted shipper probe 2 is cleaned. The used distilled water discharged into the cleaning tank 5 is delivered, as a waste liquid, from a bottom portion of the cleaning tank 5 to the waste liquid bottle 13 via the conduit P11.

Next, the shipper probe 2 is moved upward to move the tip end portion thereof out of the cleaning tank 5, and then the shipper probe 2 is horizontally moved to above the buffer solution bottle 3 and is further moved downward to insert the tip end portion thereof into the buffer solution in the buffer solution bottle 3. Next, the buffer solution in the buffer solution bottle 3 is aspirated by the syringe 11, and 1000 µl of the buffer solution is introduced into the flow chamber 17. By the introduction of the buffer solution, the unreacted second reagent remaining in the flow chamber 17 is washed away. At this time, the buffer solution used for cleaning and the unreacted reagent are aspirated into the syringe 11 from the flow chamber 17 via the conduit P3, the first pinch valve 7, and the conduit P4.

By the above operations, the reaction product and the unreacted first reagent (magnetic particles) are captured above the working electrode 15, in the flow chamber 17, and the surrounding of the reaction product and the unreacted first reagent, that is, the entire flow chamber 17, is filled with the buffer solution containing the TPA used for inducing the excitation of the labeling substance. On the other hand, after the first pinch valve 7 is closed, the buffer solution and the unreacted reagent aspirated into the syringe 11 are discharged into the waste liquid bottle 13 by the syringe 11 by opening the second pinch valve 8.

After the above process is completed, a voltage based on a sequence determined between the working electrode 15 and the counter electrodes 16A and 16B disposed on both sides of the working electrode 15 on the same plane is applied, and the following reactions (1) to (5) are performed.
(1)

   TPA → TPA⁺ + e⁻
(2)

   TPA+ → TPA* + H⁺
(3)

   Ru(bpy)₃²⁺ → Ru(bpy)₃³⁺ + e⁻
(4)

   Ru(bpy)₃³⁺ + TPA* → Ru(bpy) ₃^{2+*}
(5)

   Ru(bpy)₃^{2+*} → Ru(bpy) ₃²⁺ + photon (620 nm)

That is, the TPA in the buffer solution is oxidized by application of a voltage, and Ru (bpy) ₃²⁺ that is the labeling substance in the reaction product emits light. The light generated by this reaction is introduced to a photoelectric surface of the photomultiplier tube 19 through the transparent light receiving window 22 provided on the flow chamber 17, and an amount of luminescence thereof is measured. The amount of luminescence is compared with an amount of luminescence of the time when a control substance having a known TSH concentration is measured, and a TSH concentration in the sample is calculated.

In the above reaction process, in order to reduce an influence of the magnetic field on a multiplication efficiency of the photomultiplier tube, the magnet 24 disposed for a purpose of capturing, above the working electrode 15, the reaction product formed by binding to the magnetic particles is moved to the retracted position, where the influence of the magnetic field is not exerted on a surface of the working electrode 15, by driving the stepping motor 26 immediately before or possibly immediately after the luminescence is performed by an electrochemical reaction caused by the application of the voltage.

After a luminescence reaction is completed, the inside of the flow chamber 17 is cleaned. First, the first pinch valve 7 is opened, the second pinch valve 8 and the third pinch valve 9 are closed, the shipper probe 2 whose tip end is cleaned with the distilled water in advance in the same manner as described above is horizontally moved to above the cleaning liquid bottle 4 and then moved downward, the tip end portion of the shipper probe 2 is inserted into the cleaning liquid in the cleaning liquid bottle 4, and in this state, the suction of the cleaning liquid in the cleaning liquid bottle 4 by the syringe 11 is started. At this time, for a purpose of increasing a cleaning efficiency, it is preferable to alternately aspirate the cleaning liquid and air by fixed amounts by moving the shipper probe 2 up and down while the cleaning liquid is being aspirated by the syringe 11. The cleaning liquid thus aspirated is guided into the flow chamber 17 through the conduit P2, and washes away the buffer solution, the reaction product, and the unreacted first reagent (magnetic particles) after completion of the reaction that are remained in the flow chamber 17. Thereafter, the waste liquid and the cleaning liquid aspirated into the syringe 11 are discharged into the waste liquid bottle 13 by closing the first pinch valve 7, opening the second pinch valve 8, and squeezing out the syringe 11.

After the above process is completed, the second pinch valve 8 is closed and the first pinch valve 7 is opened again, 1000 µl of the buffer solution containing the TPA is aspirated from the inside of the buffer solution bottle 3 using the syringe 11 by the shipper probe 2 whose tip end is cleaned with the distilled water in advance, and the cleaning liquid remaining in the conduit P2 and the flow chamber 17 is washed away, and then the inside of the conduit P2 and the flow chamber 17 is replaced with the buffer solution. By this operation, measurement of the TSH with respect to one sample is completed.

### [Flow Cell]

Next, a configuration of the flow cell 6 will be described in more detail with reference to Figs. 2 to 4. As described above, the base 18 constituting the flow cell 6 is provided with the inlet (flow path inlet) 35 and the outlet (flow path outlet) 36 of the reaction solution. In order to prevent, as much as possible, adhesion of dirt due to protein components or the like in the sample and deterioration due to the cleaning liquid or the like, the base 18 is made of, for example, an electrically non-conductive material such as a fluorine resin such as polytetrafluoroethylene, a polyether ether resin, or an acrylic resin.

The light receiving window 22 is made of a non-conductive and transparent material. The light receiving window 22 does not necessarily need to be a transparent material as long as light having a predetermined wavelength emitted in the flow cell is transmitted therethrough. For example, the acrylic resin can be used.

Examples of the material of the working electrode 15 and the counter electrodes 16A and 16B include gold, platinum, palladium, tungsten, iridium, nickel, and an alloy thereof, a carbon material, and the like.

In addition, for example, a material may be used that is obtained by plating a material such as gold, platinum, palladium, tungsten, iridium, nickel, or an alloy thereof, or a carbon material on a base material such as titanium, and forming a film by sputtering or the like.

The gasket 18A is disposed between the base 18 and the light receiving window 22, and is one of components forming the flow chamber 17. Examples of the material of the gasket 18A include a fluorine resin such as polytetrafluoroethylene, and a rubber such as a silicon rubber and a fluoro rubber. Particularly, it is preferable to use the fluoro rubber from viewpoints of chemical resistance and sealing property of the flow cell 6.

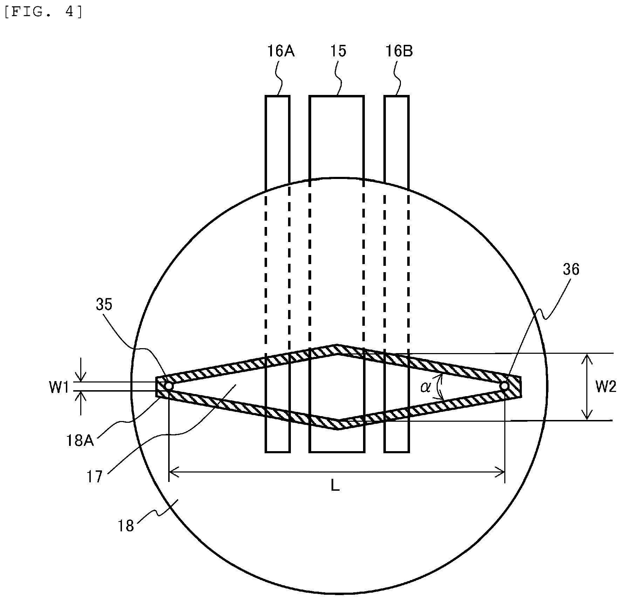

The inventor has diligently studied the material of the gasket 18A capable of stabilizing the flow path shape of the flow cell 6, that is, the shape of the flow chamber 17 over a long period of time. Fig. 4 is a sectional view taken along a line II-II in Fig. 3. By repeatedly using the flow cell 6, a maximum width W2 of the flow chamber 17 decreases, which is caused by swelling of the gasket 18A due to contact with various liquid chemicals. Therefore, it is preferable to apply a material having high chemical resistance to the gasket 18A.

On the other hand, a surface of the gasket 18A made of a fluoro rubber has adhesion. It is considered that, due to the surface adhesion, a force acts against a force of reducing the maximum width W2 due to the swelling of the gasket 18A, between upper and lower surfaces of the gasket 18A and the base 18 and the light receiving window 22.

Therefore, it is considered that the surface adhesion is an important index for selecting the material of the gasket 18A.

Therefore, a plurality of types of fluoro rubbers were used as the material of the gasket 18A, and the chemical resistance, the surface adhesion, and long-term shape stability thereof were evaluated. The evaluated test samples of fluoro rubber included three types, that is, binary FKM: vinylidene fluoride/hexafluoropropylene (Sample No. 1), ternary FKM: vinylidene fluoride/hexafluoropropylene/tetrafluoroethylene (Sample Nos 2 to 4), and FEPM: tetrafluoroethylene/propylene (Sample Nos 5 to 7), which are often used as the material of gasket. The FKM and the FEPM are fluoro rubbers classified by American Society for Testing and Materials (ASTM) D1418 standard. A type A durometer hardness (in accordance with Japanese Industrial Standards (JIS) K6253) of Sample No. 1 is 70 HS, hardnesses of Sample Nos 2 to 4 are 60 HS to 80 HS, and hardnesses of Sample Nos 5 to 7 are 60 HS to 80 HS. The types of the fluoro rubber in the test sample are shown in Table 1 described later.

The flow chamber 17 having a spindle shape shown in Fig. 4 was formed using the gasket 18A made of the fluoro rubbers described above. A minimum width W1 at both ends of the flow chamber 17 having the spindle shape was 1 mm, the maximum width W2 at a center was 5 mm, a length L was 33 mm, an opening angle α was 16.2°, and a thickness was 0.5 mm. A polyether ether resin was used for the base 18. The flow path inlet 35 and the flow path outlet 36 provided in the base 18 have a size of 1 mm in diameter. An acrylic resin (thickness: 4 mm), which is a non-conductive material having a light transmittance of 90% or more, was used for the light receiving window 22. Platinum was used for the working electrode 15 and the counter electrodes 16A and 16B. An area of the working electrode 15 exposed to the flow chamber 17 was about 23.2 mm². The counter electrodes 16A and 16B were disposed at an interval of 2 mm from the working electrode 15.

The chemical resistance and the surface adhesion of the gasket 18A made of the materials described above were evaluated.

First, a method of evaluating the chemical resistance of the gasket 18A will be described. The gasket 18A prepared in the shape shown in Fig. 4 was immersed in a 0.2 mol/L of potassium hydroxide solution in an environment of 40°C for 31 days, and an amount of dimensional change was evaluated (chemical immersion test). An evaluated dimension is a dimension corresponding to the maximum width W2 of the flow chamber 17. It means that in this evaluation, the smaller the amount of dimensional change, the higher the chemical resistance.

It is preferably that the amount of dimensional change evaluated by the chemical resistance test described above is 0.2 mm or less.

If the amount of dimensional change exceeds 0.2 mm, a liquid flow, in the vicinity of the working electrode 15, in the flow chamber 17 changes from an initial state, which affects analysis performance. It is more preferably that the amount of dimensional change is 0.1 mm or less, and it is still more preferably that the amount of dimensional change is 0.03 mm or less.

Next, a method of evaluating the surface adhesion of the gasket 18A will be described. A test piece having a size of 10 mm square and a thickness of 0.5 mm was prepared from the materials of the gasket 18A. A lower surface of the test piece was fixed to an acrylic resin A (20 mm × 30 mm, thickness 2 mm) with a double-sided tape. An acrylic resin B (20 mm × 60 mm, thickness 2 mm) was placed on an upper surface of the test piece, a 2 kg weight was further allowed to stand on the acrylic resin B for 1 minute, and the acrylic resin A, the test piece, and the acrylic resin B were integrated into an evaluation sample. The acrylic resin B of the evaluation sample was moved in a vertical direction, and a force at which an interface between the test piece and the acrylic resin B separates was measured with a force gauge, and a measured value was defined as a surface adhesive force (N/cm²) .

It is preferably that the surface adhesive force evaluated by the surface adhesion evaluation test described above is 14 N/cm² or more and 40 N/cm² or less. When the surface adhesive force exceeds 40 N/cm², the light receiving window 22 and the gasket 18A are strongly adhered to each other, and handling is difficult in a manufacturing process of the flow cell 6. On the other hand, when the surface adhesive force is less than 14 N/cm², the sealing property of the flow chamber 17 is reduced, and a leakage risk of the solution is increased. It is more preferably that the surface adhesive force is 15 N/cm² or more and 40 N/cm² or less, and it is still more preferably that the surface adhesive force is 15 N/cm² or more and 17 N/cm² or less.

Next, a method of evaluating the long-term shape stability of the gasket 18A will be described. Only a cleaning process of the automatic analysis device was repeatedly performed by 100,000 times with respect to the flow cell 6, an area for the working electrode 15 portion of the flow chamber 17 was measured, and a variation amount with respect to an initial area was evaluated. The area variation amount being small means that the material of the gasket 18A is excellent in the long-term shape stability.

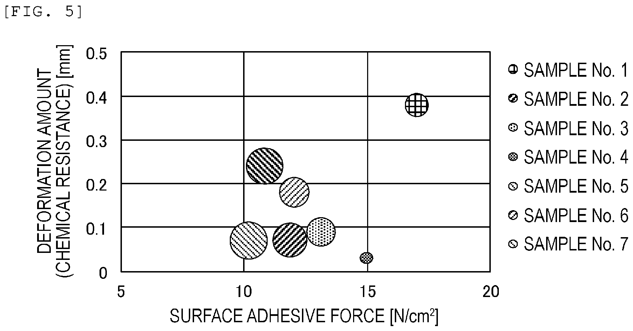

The deformation amounts, the surface adhesive forces, and the area variation amounts for Sample Nos 1 to 7 are shown in the following Table 1. Fig. 5 is a graph in which the deformation amounts (chemical resistance) and the surface adhesive forces of the test samples are plotted.

A size of a plot indicates the area variation amount.

**[Table 1]**

| Sample NO. | Gasket material | | | Hardness [HS] | Deformation amount [mm] | Surface adhesive force [N/cm²] | Area variation amount [mm²] |
|---|---|---|---|---|---|---|---|
| 1 | FKM | Binary | Vinylidene fluoride/hexafluoropropylene | 70 | 0.38 | 17.0 | 0.20 |
| 2 | FKM | Ternary | Vinylidene fluoride/hexafluoropropylene/tetrafluoroethylene | 60 | 0.07 | 11.9 | 0.41 |
| 3 | | | | 70 | 0.09 | 13.1 | 0.30 |
| 4 | | | | 80 | 0.03 | 15.0 | 0.05 |
| 5 | FEPM | Binary | Tetrafluoroethylene/propylene | 60 | 0.07 | 10.2 | 0.49 |
| 6 | | | | 70 | 0.18 | 12.0 | 0.30 |
| 7 | | | | 80 | 0.24 | 10.8 | 0.47 |

Regarding Sample No. 4, the deformation amount in the chemical immersion test was 0.2 mm or less, the surface adhesive force was 14 N/cm² or more and 40 N/cm² or less, and the area variation amount in a long-term shape stability evaluation was the smallest. That is, it was shown that the material of the gasket 18A with little long-term shape change can be selected by evaluating with two indexes of the deformation amount in the chemical immersion test and the surface adhesive force.

An analysis of a TSH containing sample was performed before and after the long-term shape stability evaluation, and as a result thereof, in the case where Sample No. 4 was used, a variation of an analysis value was the smallest and long-term stability of the analysis value was excellent.

As described above, it is shown that, according to the invention, it is possible to provide a flow cell and an automatic analysis device which are excellent in the long-term stability of the flow path shape of the flow cell and can improve the reproducibility of the analysis.

The invention is not limited to the above-mentioned embodiments, and includes various modifications.

The embodiment described above has been described for easy understanding of the invention, and is not necessarily limited to those including all the configurations described above. In addition, a part of a configuration of a certain embodiment may be replaced with a configuration of another embodiment, and a configuration of a certain embodiment may also be added with a configuration of another embodiment. A part of a configuration of each embodiment may be added, deleted, or replaced with another configuration.

### Reference Signs List

- 100:: automatic analysis device
- 1:: vessel
- 2:: shipper probe
- 3:: buffer solution bottle
- 4:: cleaning liquid bottle
- 5:: cleaning tank
- 6:: flow cell
- 7:: first pinch valve
- 8:: second pinch valve
- 9:: third pinch valve
- 10:: light detection unit
- 11:: syringe
- 12:: pump
- 13:: waste liquid bottle
- 14:: distilled water bottle
- 15:: working electrode
- 16A, 16B:: counter electrode
- 17:: flow chamber
- 18:: base
- 18A:: gasket
- 18B:: recess
- 19:: photomultiplier tube
- 20:: shield tube
- 21:: PMT case
- 22:: light receiving window
- 23:: cell holder
- 24:: magnet
- 25:: magnet holder
- 25A:: lever
- 26:: stepping motor
- 27:: socket
- 28:: fulcrum
- 30:: sampling probe
- 31:: sample bottle
- 32:: bead bottle
- 33:: first reagent bottle
- 34:: second reagent bottle
- 35:: flow path inlet
- 36:: flow path outlet
- 37, 38:: nipple
- P1 to P11:: conduit

## Claims

1. A flow cell comprising:
a base provided with an inlet and outlet of a flow path of a fluid containing an analyte,
an electrode for applying a voltage to the analyte,
a light receiving window made of a member that transmits light emitted from the analyte by the voltage applied on the electrode,
a gasket provided between the base and the light receiving window, and
a flow chamber surrounded by the base, the light receiving window and the gasket, wherein
the gasket has a deformation amount of 0 mm or more and 0.2 mm or less in the chemical immersion test defined in the following (1), and a surface adhesive force of 14 N/cm² or more and 40 N/cm² or less in the surface adhesion evaluation test defined in the following (2):
(1) Chemical immersion test: After immersion in 0.2 mol/L of potassium hydroxide solution at 40 °C for 31 days, the amount of dimensional change is measured.
(2) Surface adhesion evaluation test: A lower surface of a test piece (10 mm square, 0.5 mm thick) is fixed to acrylic resin 1 (20 mm × 30 mm, 2 mm thick) with double-sided tape; acrylic resin 2 (20 mm × 60 mm, 2 mm thick) is placed on an upper surface of the test piece; a 2 kg weight is allowed to stand on the upper surface of the acrylic resin 2 for 1 minute; the acrylic resin 2 of the evaluation sample obtained by integrating the acrylic resin 1, the test piece, and the acrylic resin 2 is moved in the vertical direction; and the force at which the interface between the test piece and the acrylic resin 2 separates is measured with a force gauge.

2. The flow cell according to claim 1, wherein the deformation amount is 0.1 mm or less.

3. The flow cell according to claim 1, wherein the deformation amount is 0.03 mm or less.

4. The flow cell according to claim 1, wherein the surface adhesive force is 15 N/cm² or more and 40 N/cm² or less.

5. The flow cell according to claim 1, wherein the surface adhesive force is 15 N/cm² or more and 17 N/cm² or less.

6. The flow cell according to any one of claims 1 to 5, wherein the material of the gasket is fluoro rubber.

7. The flow cell according to claim 6, wherein the material of the gasket is a ternary fluoro rubber composed of hexafluoropropylene/vinylidene fluoride/tetrafluoroethylene.

8. An automatic analysis device comprising:
a flow cell including a base provided with an inlet and outlet of a flow path of a fluid containing an analyte, an electrode for applying a voltage to the analyte, a light receiving window made of a member that transmits light emitted from the analyte by the voltage applied on the electrode, a gasket provided between the base and the light receiving window, and a flow chamber surrounded by the base, the light receiving window, and the gasket;
a unit for supplying a fluid containing magnetic particles having a labeling substance to the flow cell;
a voltage applying unit for applying a voltage to the electrode;
a magnetic trapping unit for trapping the magnetic particles supplied to the flow cell at a predetermined location by a magnetic field; and
a light detection unit for detecting light generated from the flow cell via the light receiving window, wherein
the gasket has a deformation amount of 0 mm or more and 0.2 mm or less in the chemical immersion test defined in the following (1), and the gasket has a surface adhesive force of 14 N/cm² or more and 40 N/cm² or less in the surface adhesion evaluation test defined in the following (2):
(1) Chemical immersion test: After immersion in 0.2 mol/L of potassium hydroxide solution at 40 °C for 31 days, the amount of dimensional change is measured.
(2) Surface adhesion evaluation test: A lower surface of a test piece (10 mm square, 0.5 mm thick) is fixed to acrylic resin 1 (20 mm × 30 mm, 2 mm thick) with double-sided tape; acrylic resin 2 (20 mm × 60 mm, 2 mm thick) is placed on an upper surface of the test piece; a 2 kg weight is allowed to stand on the upper surface of the acrylic resin 2 for 1 minute; the acrylic resin 2 of the evaluation sample obtained by integrating the acrylic resin 1, the test piece, and the acrylic resin 2 is moved in the vertical direction; and the force at which the interface between the test piece and the acrylic resin 2 separates is measured with a force gauge.

9. The automatic analysis device according to claim 8, wherein the deformation amount is 0.1 mm or less.

10. The automatic analysis device according to claim 8, wherein the deformation amount is 0.03 mm or less.

11. The automatic analysis device according to claim 8, wherein the surface adhesive force is 15 N/cm² or more and 40 N/cm² or less.

12. The automatic analysis device according to claim 8, wherein the surface adhesive force is 15 N/cm² or more and 17 N/cm² or less.

13. The automatic analysis device according to any one of claims 8 to 12, wherein the material of the gasket is fluoro rubber.

14. The automatic analysis device according to claim 8, wherein the material of the gasket is a ternary fluoro rubber composed of hexafluoropropylene/vinylidene fluoride/tetrafluoroethylene.
